# EUROPÄISCHE PATENTSCHRIFT

(11) **EP 0 458 109 B1**
(45) Veröffentlichungstag und Bekanntmachung des Hinweises auf die Patenterteilung: **13.07.1994**
(21) Anmeldenummer: 91107253.6
(22) Anmeldetag: 04.05.1991
(51) Int. Cl.: C07D 213/61, C07D 213/26, C07D 239/30

(54) **Seitenkettenchlorierung von alkylierten Pyridin- oder Pyrimidin-Derivaten**
Side chain chlorination of alkylated pyridine or pyrimidine derivatives
Chloration de la chaîne latérale de dérivés de pyridines ou pyrimidines alkylés

(30) Priorität: 19.05.1990 DE 4016175
(43) Veröffentlichungstag der Anmeldung: 27.11.1991
(73) Patentinhaber: BAYER AG, 51368 Leverkusen (DE)
(72) Erfinder: Günther, Andreas, Dr., W-5000 Köln 80 (DE)

(56) Entgegenhaltungen:
- EP-A- 0 012 108
- EP-A- 0 260 485
- DE-A- 1 470 064

## Beschreibung

Es ist bekannt, die Seitenketten von alkylierten Stickstoff-Heteroaromaten, insbesondere von alkylierten Pyridinen, mit Chlor umzusetzen, wobei ein oder mehrere Wasserstoffatome durch Chloratome substituiert werden. Es ist ferner bekannt, die Geschwindigkeit und Ausbeute dieser Reaktion durch radikalbildende Strahlung oder durch chemische Radikalbildner günstig zu beeinflussen. Schließlich ist bekannt, daß die Anwendung eines Lösungsmittels für diese Reaktion oftmals günstig ist, z.B. weil das Reaktionsprodukt bei der gewählten Reaktionstemperatur fest ist oder weil das Lösungsmittel die Ausbeute erhöht

Die Auswahl dieses Lösungsmittels ist jedoch problematisch. Kohlenwasserstoffe kommen kaum in Frage, da sie in der Regel selbst mit dem eingesetzten Chlor reagieren. Chlorkohlenwasserstoffe, allem Tetrachlorkohlenstoff, wurden dagegen oft verwendet (siehe z.B. EP-A-260 485; Chemical Abstracts 84 (17): 121665p). Aus ökologischen und toxikologischen Gründen werden diese Lösungsmittel für die betriebliche Praxis jedoch in zunehmendem Maße zu Problemstoffen. Schließlich wurden auch schon anorganische Stoffe, z.B. konzentrierte Schwefelsäure oder Chlorsulfonsäure (siehe DE-A 1 470 064), als Lösungsmittel vorgeschlagen. Hier stört jedoch, daß bei der Aufarbeitung der Reaktionsmischung große Mengen stark belastetes Abwasser anfallen.

Es bestand daher ein Bedarf, für die genannte Reaktion ein chlorfreies Lösungs- mittel zu finden, welches die Seitenkettenchlorierung von Stickstoffheteroamaten ähnlich günstig wie Tetrachlorkohlenstoff beeinflußt und sich nach der Reaktion in hohem Maße durch einfache Destillation zurückgewinnen läßt. Dabei sollten die oben geschilderten Nachteile der bisher bekannt gewordenen Verfahren vermieden werden.

Überraschenderweise wurde nun gefunden, daß man bei der Chlorierung der Seitenketten von gegebenenfalls substituierten alkylierten Pyridin- oder Pyrimidin-Derivaten mit elementarem Chlor besonders günstige Ergebnisse erzielt, wenn man Acetonitril als Lösungsmittel verwendet und die Umsetzung in Gegenwart radikalbildender Strahlung oder von chemischen Radikalbildnern durchführt.

Überraschend ist dies sowohl deshalb, weil die Methylgruppe des Acetonitrils erwartungsgemäß ebenso wie die Alkyl-Seitenkette des Stickstoff-Heteroaromaten mit Chlor reagieren sollte (siehe z.B. D-E-A 227 134, US-A 3 825 581 oder US-A 3 418 228), als auch deshalb, weil die Vergleichsversuche 5 bis 8 zeigen, daß Acetonitril bei der Chlorierung der Alkyl-Seitenkette von anderen Aromaten ein weit ungünstigeres Lösungsmittel als z.B. Tetrachlorkohlenstoff ist.

Der Vorteil des erfindungsgemäßen Verfahrens besteht darin, daß man ein Lösungsmittel einsetzt, das gegenüber den chlorierten Kohlenwasserstoffen, z.B. Tetrachlorkohlenstoff, hinsichtlich Ökologie und Kanzerogenität weit günstiger ist und das in bezug auf Reaktionsgeschwindigkeit, Ausbeute und Wiedergewinnbarkeit ähnliche, zum Teil sogar bessere Eigenschaften als Tetrachlorkohlenstoff aufweist (siehe dazu Vergleichsbeispiele 2 bis 4). Auch gegenüber der Möglichkeit, die Reaktion lösungsmittelfrei durchzuführen, bestehen große Vorteile, wie Vergleichsbeispiel 1 zeigt.

Zur Durchführung des erfindungsgemäßen Verfahrens arbeitet man bevorzugt mit einer Lösung in Acetonitril, welche 5 bis 70 Gew.-% des zu chlorierenden alkylierten Stickstoff-Heteroaromaten enthält.

Als alkylierte Stickstoff-Heteroaromaten kommen vorzugsweise alkylierte, gegebenenfalls weitere Substituenten enthaltende Pyridin- oder Pyrimidinderivate in Betracht. Beispielhaft seien genannt: 3-Methylpyridin, 2-Chlor-5-methyl pyridin, 2,4-Dichlor-6-methyl-pyridin, 2,4-Dichlor-6-methyl-pyrimidin, 2-Ethyl-pyridin. Insbesondere bevorzugt wird 2-Chlor-5-methyl-pyridin beim erfindungsgemäßen Verfahren als Ausgangskomponente eingesetzt.

Die Reaktionstemperatur des erfindungsgemäßen Verfahrens beträgt bevorzugt 50°C bis 150°C und der Reaktionsdruck 0,3 bis 5,0 bar. Besonders bevorzugt stimmt man Temperatur und Druck so ab, daß sich Rückflußbedingungen einstellen.

Man kann die Reaktion durch Variation der Bedingungen, z.B. Einleitungszeit des Chlorgases so steuern, daß Einfach- oder Mehrfachchlorierung der Alkyl-Seitenkette eintritt.

Das erfindungsgemäße Verfahren arbeitet bevorzugt bei Anwesenheit von radikalbildender Strahlung, wie z.B. UV-Licht, oder von radikalbildenden Substanzen, wie z.B. Azobiscarbonsäurenitrilen oder Diacylperoxiden. Wenn das Zielmolekül eine Monochlormethylverbindung ist, arbeitet man besonders bevorzugt mit radikalbildenden Substanzen, da überraschend festgestellt wurde, daß sie in diesem Fall dem UV-Licht deutlich überlegen sind (siehe Beispiel 1 und 2).

Die Bestimmung des Endpunktes der Reaktion geschieht mit herkömmlichen Methoden, z.B. analysiert man die Reaktionsmischung während der Reaktion gaschromatographisch und unterbricht die Chlorzufuhr, sobald der Gehalt an dem gewünschten Produkt sein Maximum erreicht hat. Dabei kann es auch vorteilhaft sein, die Reaktion vor diesem Maximum abzubrechen, das nicht umgesetzte Ausgangsprodukt zurückzugewinnen und erneut in die Reaktion einzusetzen.

Die Aufarbeitung der Reaktionsmischung erfolgt nach herkömmlichen Methoden, z.B. destilliert man das Acetonitril im Vakuum ab und setzt es erneut in die Reaktion ein. Hier werden Wiedergewinnungsraten von 95 % bis 98 % erreicht. Anschließend stellt man den Destillationssumpf bei Raumtemperatur mit Natronlauge neutral. Die organische Phase wird abgetrennt und das Produkt im Vakuum durch Rektifikation isoliert Der Vorlauf der Rektifikation enthält gegebenenfalls noch Ausgangsprodukt, das recyclisiert werden kann. Durch die folgenden Beispiele wird das erfindungsgemäße Verfahren weiter erläutert

### Beispiel 1

### Herstellung von 2-Chlor-5-chlormethylpyridin

1 Mol 2-Chlor-5-methylpyridin und 128 g Acetonitril werden bei Normaldruck zum Rückfluß erhitzt Man gibt 0,2 g Azobisisobuttersäurenitril als Radikalbildner zu und wiederholt diese Zugabe jede halbe Stunde. Gleichzeitig leitet man Chlorgas in leichtem Überschuß ein und hält die Reaktionsmischung weiterhin ständig am Rückfluß. Nach 4 Stunden bricht man die Reaktion ab. Bei etwa 85°C Sumpftemperatur destilliert man das Lösungsmittel durch langsames Anlegen von Vakuum ab. Man gewinnt 122 g zurück, die in den nächsten Ansatz zurückgeführt werden können. Aus dem Sumpf isoliert man 0,37 Mol Produkt und 0,53 Mol Ausgangsprodukt. Bezogen auf das umgesetzte Ausgangsprodukt (0,47 Mol) ergibt sich damit eine Ausbeute von 79 %.

### Vergleichsbeispiel 1

zeigt, daß die lösungsmittelfreie Herstellung von 2-Chlor-5-chlormethylpyridin ungünstiger ist:

In 1 Mol 2-Chlor-5-methylpyridin wird bei 85°C Chlorgas in leichtem Überschuß eingeleitet. Anfangs und danach jede halbe Stunde gibt man 0,2 g Azoisobuttersäurenitril zu. Nach 4 Stunden sind lediglich 0,2 Mol Produkt entstanden.

### Vergleichsbeispiel 2

zeigt die Herstellung von 2-Chlor-5-chlormethylpyridin in Tetrachlorkohlenstoff:

1 Mol 2-Chlor-5-methylpyridin und 128 g Tetrachlorkohlenstoff werden zum Rückfluß erhitzt. Man gibt 0,2 g Azobisisobuttersäurenitril zu und wiederholt diese Zugabe jede halbe Stunde. Gleichzeitig leitet man Chlorgas in leichtem Überschuß ein. Nach 3 Stunden bricht man die Reaktion ab und isoliert 0,37 Mol Produkt und 0,53 Mol Ausgangsprodukt.

### Beispiel 2

### Herstellung von 2-Chlor-5-chlormethylpyridin

1 Mol 2-Chlor-5-methylpyridin und 128 g Acetonitril werden zum Rückfluß erhitzt. Man bestrahlt mit UV-Licht und leitet Chlorgas in leichtem Überschuß ein. Die Reaktion wird abgebrochen, sobald 0,37 Mol Produkt entstanden sind. Gleichzeitig werden 0,46 Mol Ausgangsprodukt zurückgewonnen. Bezogen auf das umgesetzte Ausgangsprodukt ergibt sich eine Ausbeute von 69 %.

### Beispiel 3

### Herstellung von 2,4-Dichlor-6-chlormethylpyrimidin

1 Mol 2,4-Dichlor-6-methylpyrimidin und 326 g Acetonitril werden zum Rückfluß erhitzt. Man gibt 0,12 g Azobisisobuttersäurenitril zu und wiederholt diese Zugabe jede halbe Stunde. Gleichzeitig leitet man Chlorgas in leichtem Überschuß ein. Nach 0,75 Stunden bricht man die Reaktion ab und erhält nach gaschromatographischer Analyse 0,51 Mol Produkt neben 0,31 Mol Ausgangsprodukt (Ausbeute 74 %).

### Vergleichsbeispiel 3

zeigt die Herstellung von 2,4-Dichlor-6-chlormethylpyrmidin in Tetrachlorkohlenstoff:

1 Mol 2,4-Dichlor-6-methylpyrimidin und 326 g Tetrachlorkohlenstoff werden zum Rückfluß erhitzt. Man gibt 0,12 g Azobisisobuttersäurenitril zu und wiederholt diese Zugabe jede halbe Stunde. Gleichzeitig leitet man Chlorgas in leichtem Überschuß ein. Nach 1,1 Stunden bricht man die Reaktion ab und erhält 0,51 Mol Produkt neben 0,31 Mol Ausgangsprodukt.

### Beispiel 4

### Herstellung von 2,4-Dichlor-6-dichlormethylpyrimidin

Man arbeitet wie in Beispiel 3, beendet die Reaktion jedoch erst nach 3 Stunden. Man erhält 0,71 Mol Produkt neben 0,19 Mol 2,4-Dichlor-6-chlormethylpyrimidin. Letzteres kann in den Prozeß zurückgeführt werden, so daß sich eine Ausbeute von 88 % ergibt.

### Vergleichsbeispiel 4

zeigt die Herstellung von 2,4-Dichlor-6-dichlormethylpyrimidin in Tetrachlorkohlenstoff:

Man arbeitet wie in Vergleichsbeispiel 3, beendet die Reaktion aber erst nach 3,75 Stunden. Man erhält 0,71 Mol Produkt neben 0,19 Mol 2,4-Dichlor-6-chlormethylpyrimidin.

Vergleichsbeispiele 5 bis 8 stellen die Lösungsmittel Acetonitril und Tetrachlorkohlenstoff bei der Seitenkettenchlorierung von Aromaten ohne Stickstoff gegenüber.

### Vergleichsbeispiel 5

### Herstellung von 2,4-Dichlor-chlormethylbenzol

1 Mol 2,4-Dichlor-methylbenzol und 322 g Tetrachlorkohlenstoff werden zum Rückfluß erhitzt. Man gibt 0,8 g Azobisisobuttersäurenitril zu und leitet Chlorgas in leichtem Überschuß ein. Nach 0,5 Stunden erhält man nach gaschromatographischer Analyse 0,75 Mol Produkt.

### Vergleichsbeispiel 6

### Herstellung von 2,4-Dichlor-chlormethylbenzol

1 Mol 2,4-Dichlor-methylbenzol und 322 g Acetonitril werden zum Rückfluß erhitzt. Man gibt 0,8 g Azobisisobuttersäurenitril zu und wiederholt diese Zugabe jede halbe Stunde. Gleichzeitig leitet man Chlorgas in leichtem Überschuß ein. Erst nach 2 Stunden erhält man 0,75 Mol Produkt.

### Vergleichsbeispiel 7

### Herstellung von 1,4-Bis-trichlormethylbenzol

1 Mol 1,4-Dimethylbenzol und 1.000 g Tetrachlorkohlenstoff werden zum Rückfluß erhitzt.. Man gibt 0,2 g Azobisisobuttersäurenitril zu und wiederholt diese Zugabe jede halbe Stunde. Gleichzeitig leitet man Chlorgas in leichtem Überschuß ein. Nach 18 Stunden unterbricht man die Reaktion und kann 0,9 Mol Produkt isolieren.

### Vergleichsbeispiel 8

### Herstellung von 1,4-Bis-trichlormethylbenzol

Man arbeitet wie in Vergleichseispiel 7, jedoch mit 1.000 g Acetonitril anstelle von Tetrachlorkohlenstoff. Nach 18 Stunden Reaktionszeit ist noch keinerlei Produkt entstanden.

## Patentansprüche

1. Verfahren zur Chlorierung der Seitenketten von gegebenenfalls substituierten alkylierten Pyridin- oder Pyrimidin-Derivaten mit elementarem Chlor, dadurch gekennzeichnet, daß man als Lösungsmittel Acetonitril verwendet und die Umsetzung in Gegenwart radikalbildender Strahlung oder von chemischen Radikalbildnern durchführt.

2. Verfahren gemäß Anspruch 1, dadurch gekennzeichnet, daß man Temperatur und Druck so abstimmt, daß sich während der Reaktion Rückflußbedingungen einstellen.

3. Verfahren gemäß Anspruch 1, dadurch gekennzeichnet, daß als chemischer Radikalbildner ein Azobiscarbonsäurenitril oder ein Diacylperoxid verwendet wird.

4. Verfahren gemäß Anspruch 1 bis 3, dadurch gekennzeichnet, daß 2-Chlor-5-methyl-pyridin zu 2-Chlor-5-chlormethyl-pyridin umgesetzt wird.

## Claims

1. Process for the chlorination of the side chains of optionally substituted alkylated pyridine or pyrimidine derivatives with elemental chlorine, characterized in that acetonitrile is used as a solvent and the reaction is carried out in the presence of free radical-initiating radiation or of chemical free radical initiators.

2. Process according to Claim 1, characterized in that temperature and pressure are adjusted such that reflux conditions are established during the reaction.

3. Process according to Claim 1, characterized in that an azobiscarbonitrile or a diacyl peroxide is used as the chemical free radical initiator.

4. Process according to Claims 1 to 3, characterized in that 2-chloro-5-methylpyridine is reacted to give 2-chloro-5-chloromethylpyridine.

## Revendications

1. Procédé pour la chloruration des chaînes latérales de dérivés alkylés, éventuellement substitués, de la pyridine ou de la pyrimidine par le chlore élémentaire, caractérisé en ce que l'on utilise en tant que solvant l'acétonitrile et on effectue la réaction sous un rayonnement formant des radicaux libres ou en présence d'inducteurs radicalaires chimiques.

2. Procédé selon la revendication 1, caractérisé en ce que l'on règle mutuellement la température et la pression de manière à réaliser la réaction au reflux.

3. Procédé selon la revendication 1, caractérisé en ce que l'on utilise en tant qu'inducteur radicalaire chimique un azo-bis-carboxynitrile ou un peroxyde de diacyle.

4. Procédé selon les revendications 1 a 3, caractérisé en ce que l'on convertit la 2-chloro-5-méthylpyridine en la 2-chloro-5-chlorométhylpyridine.
